# EUROPÄISCHE PATENTSCHRIFT

(11) **EP 2 509 336 B1**
(45) Veröffentlichungstag und Bekanntmachung des Hinweises auf die Patenterteilung: **24.08.2016**
(21) Anmeldenummer: 12163036.2
(22) Anmeldetag: 03.04.2012
(51) Int. Cl.: H04Q 9/00, H02M 3/07, G01N 33/00

(54) **Sensorvorrichtung zum Melden von vorhandenem Gas**
Sensor device for reporting the presence of gas
Dispositif de capteur d'alerte de présence de gaz

(30) Priorität: 04.04.2011 DE 102011001774
(43) Veröffentlichungstag der Anmeldung: 10.10.2012
(73) Patentinhaber: Unitronic AG, 40472 Düsseldorf (DE)
(72) Erfinder: Schäfer, Eduard, 46519 Alpen (DE); Ihln, Matthias, 40225 Düsseldorf (DE)
(74) Vertreter: Michalski Hüttermann & Partner Patentanwälte mbB

(56) Entgegenhaltungen:
- DE-A1-102007 014 398
- DE-C2- 10 150 128
- JP-A- 2010 055 356
- US-A- 5 184 500

## Beschreibung

Die Erfindung betrifft eine Sensorvorrichtung zum Melden von vorhandenem Gas und ein Verfahren zum Melden von vorhandenem Gas.

Sensorvorrichtungen zum Melden von vorhandenem Gas weisen üblicherweise ein Sensormodul zum Erfassen des vorhandenen Gases und zum Ausgeben eines Spannungssignals basierend auf dem erfassten Gas und ein Benachuchtigungsmodul zum Ausgeben eines Alarmsignals basierend auf dem ausgegebenen Spannungssignal auf. Die Sensorvorrichtung ist an eine externe Energiequelle, beispielsweise eine Batterie oder einen Netzanschluss für ein Elektrizitätsnetz, gekoppelt, so dass eine elektrische Versorgung des Benachrichtigungsmoduls gewährleistet ist. Die Umgebung der Sensorvorrichtung kann mittels einer akustischen Alarmmeldung des Benachrichtigungsmoduls über das vorhandene Gas informiert werden.

Solche Sensorvorrichtung sind je nach der Art des zu erfassenden Gases in verschiedensten Bereichen, beispielsweise für den Brandschutz, für Laborüberwachungen usw., einsetzbar.

Die bekannte Sensorvorrichtung kann jedoch unzuverlässig arbeiten, wenn eine elektrische Verbindung zwischen dem Benachuchtigungsmodul und der externen Energiequelle unterbrochen wird. Dies kann beispielsweise durch einen Ablauf einer Lebenszeit der Batterie der Sensorvorrichtung oder durch eine Unterbrechung zwischen der Sensorvorrichtung und dem Elektrizitätsnetz verursacht werden.

Ferner kann es vorkommen, dass die akustische Alarmmeldung, die mittels des Benachrichtigungsmoduls erzeugt wird, nicht gehört wird, da sich keine Personen innerhalb der akustischen Reichweite der Sensorvorrichtung befinden.

Es sind auch weitere Arten von Sensorvorrichtungen bekannt, die zum Erfassen von Umgebungseinflüssen eingerichtet sind. Es ist beispielsweise aus DE 101 50 128 C2 eine Sensorvorrichtung und ein das Sensorsystem aufweisendes Überwachungssystem zur Bestimmung einer Position von beweglichen Einrichtungen in Gebäuden oder zur Bestimmung der Beleuchtungsstärke bekannt. Die bekannte Sensorvorrichtung weist dazu einen Spanungsgenerator zum Umwandeln von nicht-elektrischen Energie in eine Spannung, einen Energiespeicher zum Speichern von Energie basierend auf der erzeugten Spannung, eine Prozessorsteuerung zum Steuern eines Senders, ein Sensorelement zum quantitativen Erfassen des Umgebungseinflusses und einen Sender zum Senden einer Benachrichtigung auf, die eine Information über den quantitativ erfassten Umgebungseinfluss in Form eines Messwerts aufweist. Eine mittels des Spanungsgenerators erzeugte Spannung kann zum Betreiben des Senders verwendet werden. Bei einer Verwendung des Sensorsystems als Beleuchtungssensorsystems können der Spanungsgenerator und das Sensorelement als identisches Bauteil, nämlich als Solarzelle, ausgebildet sein.

Ein Konzept des bekannten Sensorsystems kann allerdings nicht auf eine Sensorvorrichtung zum Melden von vorhandenem Gas übertragen werden, da das Benachuchtigungsmodul einer Sensorvorrichtung zum Melden von vorhandenem Gas eine relativ hohe Versorgungsspannung benötigt, die nicht mittels eines Sensormoduls zum Erfassen des vorhandenen Umgebungsgases erzeugbar sein kann.

Ferner kann das die Solarzelle aufweisende Beleuchtungssensorsystem nicht kontinuierlich bei Dunkelheit betrieben werden, da die Solarzelle während Dunkelheit keine Spannung an den Energiespeicher liefern und somit das Beleuchtungssystem nach Verbrauch der im Energiespeicher gespeicherten Energie ohne Spannungsversorgung ist.

US 5,184,500 beschreibt einen batteriebetriebenen Gasdetektor mit einem Gassensor, welcher mit einer Stromquelle und einer Stromversorgung gekoppelt ist. Die Stromversorgung erzeugt eine relativ hohe Spannung, während sich der Gassensor in der Aufwärmphase befindet und eine relativ niedrige Spannung, nachdem sich der Sensor erwärmt hat. Der Gassensor, welcher auf die Anwesenheit eines Gases reagiert, ist mit einem Verstärker mit einer Schaltung für eine variable Empfindlichkeit gekoppelt. Der Verstärker ist weiter mit einem Mikroprozessor verbunden, der die Konzentration des erfassten Gases bestimmt und diese visuelle auf einer Anzeige anzeigt. Der Gasdetektor kann dabei das Vorhandensein einer Vielzahl von Gasen messen. Die Gaskonzentration des erfassten Gases wird dabei durch die Größe des von einem Verstärker erzeugten Signals bestimmt.

Daher kann es eine Aufgabe der vorliegenden Erfindung sein, eine Sensorvorrichtung zum Melden von vorhandenem Gas bereitzustellen, die autark von einer Umgebungsspannung betrieben werden kann.

Diese Aufgabe wird durch eine Sensorvorrichtung zum Melden von vorhandenem Gas und ein Verfahren zum Melden von vorhandenem Gas gemäß den unabhängigen Ansprüchen gelöst. Bevorzugte Ausgestaltungen sind in den Unteransprüchen beschrieben.

Gemäß einem exemplarischen Aspekt der Erfindung wird eine Sensorvorrichtung zum Melden von vorhandenem Gas bereitgestellt, wobei die Sensorvorrichtung ein Sensormodul zum Erfassen des vorhandenen Gases und zum Ausgeben eines elektrischen Signals basierend auf dem erfassten Gas, ein Signalverstärkungsmodul zum Verstärken des ausgegebenen elektrischen Signals, ein Energiespeichermodul zum Speichern von Energie, die auf dem verstärktem elektrischen Signal basiert, und ein Sendemodul zum Senden einer Benachrichtigung basierend auf der gespeicherten Energie aufweist.

Gemäß einem weiteren exemplarischen Aspekt der Erfindung wird ein Verfahren zum Melden von vorhandenem Gas bereitgestellt, wobei das Verfahren Erfassen des vorhandenen Gases und Ausgeben eines elektrischen Signals basierend auf dem erfassten Gas mittels eines Sensormoduls, Verstärken des elektrischen Signals mittels eines Signalverstärkungsmoduls, Speichern einer Energie, die auf dem verstärkten elektrischen Signal basiert, mittels eines Energiespeichermoduls und Senden einer Benachrichtigung mittels eines Sendemoduls basierend auf der gespeicherten Energie aufweist.

Eine Benachuchtigungsempfangsvoruchtung zum Empfangen einer Benachrichtigung über ein Vorhandensein von Gas kann ein Empfangsmodul zum Empfangen der Benachrichtigung (insbesondere einen Funksignalempfänger und eine entsprechende Signalschaltung), eine Verarbeitungsmodul zum Verarbeiten der empfangenen Benachrichtigung (insbesondere einen Computer) und eine Anzeigevorrichtung zum Anzeigen der verarbeiteten Benachrichtigung (insbesondere einen Bildschirm des Computers) aufweisen.

Eine Gasmeldeanordnung zum Melden eines Vorhandenseins von Gas kann eine oben beschriebene Sensorvorrichtung zum Melden von vorhandenem Gas und eine oben beschriebene Benachrichtigungsempfangsvorrichtung zum Empfangen einer Benachrichtigung über ein Vorhandensein von Gas aufweisen.

Der Begriff "elektrisches Signal" kann insbesondere ein Spannungssignal oder ein Stromsignal, das einer elektrischen Ladung zugeordnet werden kann, bezeichnen.

Gemäß den exemplarischen Aspekten der vorliegenden Erfindung kann die Sensorvorrichtung zum Melden von vorhandenem Umgebungsgas vier separate Module aufweisen, um das Sendemodul basierend auf einem verstärkten elektrischen Signal, das auf dem erfassten Gas basiert und für den Betrieb des Sendemoduls gespeichert wird, elektrisch zu versorgen.

Insbesondere kann die Sensorvorrichtung von einer externen Energiequelle unabhängig, also autark betrieben werden, da das von dem Sensormodul erzeugte (insbesondere kleine) elektrische Signal für den Betrieb des Sendemoduls ausreichend verstärkt werden kann, so dass die elektrische Versorgung der Sensorvorrichtung unabhängig von der externen Energiequelle ausgebildet sein kann. Das Vorhandensein eines Signalverstärkungsmoduls in der Sensorvorrichtung kann insbesondere ein in sich geschlossene Schaltung der Sensorvorrichtung ermöglichen, deren Bauteile bezüglich zueinander kompakt angeordnet sein können, so dass die Sensorvorrichtung trotz eines weiteren Moduls besonders klein ausgebildet sein kann. Das Vorhandensein des zusätzlichen Signalverstärkungsmoduls kann im Gegensatz zu einem Versehen der Sensorvorrichtung mit einer zusätzlichen Solarzelle zum Umwandeln von Licht in Spannung, wie bei es dem bekannten Sensorsystem verwirklicht ist, um eine Information über den Umgebungseinfluss bereitzustellen, zu bevorzugen sein, da die Schaltung der Sensorvorrichtung einfacher ausgestaltet sein kann, da nur das Sensormodul mit dem Energiespeichermodul gekoppelt sein muss.

Insbesondere kann die Sensorvorrichtung einen zuverlässigen Betrieb ermöglichen, da unabhängig von einer Anwesenheit von Personen in der Umgebung der Sensorvorrichtung vorhandenes Umgebungsgas mittels der gesendeten Benachrichtigung gemeldet werden kann.

Insbesondere kann die modulare Bauweise der Sensorvorrichtung Herstellungs- und Wartungskosten reduzieren, da bei Ausfall eines der Module der Sensorvorrichtung das fehlerhafte Modul durch ein neues, gleichartiges Modul ersetzt werden kann.

Im Folgenden werden weitere Ausführungsformen der Sensorvorrichtung beschrieben. Diese Ausführungsformen gelten auch für das Verfahren, die Benachrichtigungsempfangsvorrichtung und die Gasmeldeanordnung.

Das Sensormodul, das Signalverstärkungsmodul und das Sendemodul können parallel zueinander geschaltet sein, wodurch ein Platzbedarf der Sensorvorrichtung im Vergleich zu einer Reihenschaltung dieser Bauteile besonders gering und daher die Sensorvorrichtung kompakt ausgestaltet sein kann. Ferner kann eine Schaltungsanordnung dieser Bauteile übersichtlich ausgebildet sein.

Die Sensorvorrichtung kann ferner ein Umschaltmodul aufweisen, das mit dem Signalverstärkungsmodul, dem Energiespeichermodul und dem Sendemodul verbunden und zum Umschalten zwischen einem Ladezustand und einem Entladezustand des Energiespeichermoduls eingerichtet sein kann. Insbesondere kann der Ladezustand des Energiespeichermoduls einer elektrisch leitenden Verbindung zwischen dem Signalverstärkungsmodul und dem Energiespeichermodul zugeordnet und ein Entladezustand des Energiespeichermoduls kann einer unterbrochenen elektrisch leitenden Verbindung zwischen dem Signalverstärkungsmodul und dem Energiespeichermodul zugeordnet sein. Insbesondere kann das Umschaltmodul das Energiespeichermodul und das Sendemodul während des Entladezustands des Energiespeichermoduls miteinander verbinden, so dass das Sendemodul mit einem der gespeicherten Energie zugeordneten elektrischen Signal (insbesondere einem Spannungssignal oder einem Stromsignal) beaufschlagt werden kann, um einen Betrieb des Sendemoduls während oder zeitlich beabstandet zu dem Entladevorgang des Energiespeichermoduls zu ermöglichen. Somit kann das verstärkte elektrische Signal zusätzlich in dem Energiespeicher gesammelt werden, um während des Entladevorgangs ein zusätzlich verstärktes elektrisches Signal dem Sendemodul zur Verfügung zu stellen.

Das Umschaltmodul kann einen Transistor und einen Thyristor aufweisen, wobei eine Anode des Thyristors über den Transistor mit dem Energiespeichermodul (insbesondere und dem Signalverstärkungsmodul) und eine Kathode des Thyristors mit dem Sendemodul verbunden sein kann. Somit kann der Transistor einen Schaltzeitpunkt des Umschaltmoduls (insbesondere über die Gate-Spannung des Transistors) steuern. Eine Verwendung des Transistors als Schaltelement kann insbesondere ein schlagartiges Umschalten zwischen dem Lade- und Entladezustand des Energiespeichermoduls ermöglichen. Die Verwendung eines Thyristors kann eine zeitlich kontinuierliche Weiterleitung eines elektrischen Signals, insbesondere eines Spanungssignals, an das Sendemodul ermöglichen, solange die Anode des Thyristors mit Strom beaufschlagt wird.

Insbesondere kann der Thyristor als Thyristortetrode ausgebildet sein, die einen ersten bipolaren und einen zweiten bipolaren Transistor aufweisen kann, die über deren Basis-Anschlüsse miteinander verschaltet sein können. Insbesondere kann ein Kollektor-Anschluss des ersten Transistors mit einem Basis-Anschluss des zweiten Transistors und ein Kollektor-Anschluss des zweiten Transistors mit einem Basis-Anschluss des ersten Transistors verbunden sein.

Das Umschaltmodul kann eine Diode aufweisen, die parallel zu dem Transistor und in Sperrrichtung geschaltet (und mit dem Thyristor, insbesondere mit der Thyristortetrode, verbunden) sein kann, so dass eine Versorgung des Energiespeichermoduls mit Strom nur über den Transistor erfolgen kann, solange der Transistor das Energiespeichermodul mit einem elektrischen Signal, insbesondere mit Spannung, auflädt. Insbesondere kann die Diode nach dem Umschalten des Energiespeichermoduls in seinen Entladezustand durch das Umschaltmodul die gespeicherte Ladung direkt zu dem Thyristor transportieren.

Das Umschaltmodul kann einen Widerstand aufweisen, der zwischen dem Transistor und dem Thyristor, insbesondere einem Gate der Thyristortetrode, angeordnet sein kann, um das Energiespeichermodul mittels des Widerstands potentialtechnisch von dem Thyristor, insbesondere von der Thyristortetrode, zu entkoppeln. Insbesondere kann der Widerstand in Kombination mit dem Transistor einen zeitabhängigen Verlauf des Schaltimpulses eines dem Sendemodul zugeführten elektrischen Signals, insbesondere eines Spannungssignals, definieren.

Das Signalverstärkungsmodul kann als Ladungspumpe oder als Spannungsaufwärtswandler ausgebildet sein. Diese Ausführungsformen des Signalverstärkungsmoduls können eine besonders einfache Verstärkung des ausgegebenen elektrischen Signals für die Beaufschlagung des Energiespeichermoduls darstellen. Insbesondere kann eine Ladungspumpe eine Verdopplung oder eine Vervielfältigung des ausgegebenen elektrischen Signals ermöglichen. Ferner kann der Spanungsaufwärtswandler die Ausgabe eines quantitativ einstellbaren elektrischen Signals, insbesondere eines Spanungssignals an das Energiespeichermodul ermöglichen. Somit kann die elektrische Versorgung des Sendemoduls entsprechend an den Stromverbrauch des Sendemoduls für eine Erzeugung der Benachrichtigung angepasst werden.

Das Signalverstärkungsmodul, insbesondere das Spannungsverstärkungsmodul, kann einen Transformator, einen Kondensator und ein (insbesondere als Transistor ausgebildetes) Schaltelement aufweisen, die resonant miteinander geschaltet sein können, um eine Aufwärtswandlung des elektrischen Signals, insbesondere des Spannungssignals, zu ermöglichen. Hierbei kann eine Sekundärspule des Transformators mit dem Kondensator verbunden sein, um dem Kondensator Ladung zuzuführen. Ferner kann eine Primärspule des Transformators ebenfalls über das Schaltelement mit dem Kondensator verbunden sein, um einen zeitlichen Schaltimpuls zu erhalten. Ferner kann der Kondensator über einen Widerstand mit einer Masse verbunden sein, und der Widerstand kann in Kombination mit dem Kondensator zeitabhängige Umschaltimpulse für das Schaltelement generieren. Die Zeitperiode ist mittels Kondensator und Widerstand einstellbar.

Das Sensormodul kann ein chemisches Sensorelement und/oder eine Mehrzahl von chemischen Sensorelementen aufweisen, wodurch herkömmliche Techniken zum Erfassen von vorhandenem Gas verwendet werden können. Insbesondere können chemische Sensorelemente dazu eingerichtet sein, geringe Mengen von Gas in einer Umgebung der Sensorvorrichtung zu erfassen, so dass die Sensorvorrichtung bereits auf geringe Mengen von vorhandenem Umgebungsgas ansprechen und somit eine besonders genaue Erfassung des vorhandenen Gases bereitstellen kann.

Das Gas kann ein für Lebewesen (insbesondere Menschen) toxisches Gas aufweisen, so dass die Sensorvorrichtung zum Melden von für die Lebewesen gesundheitlich gefährliches Gas geeignet und daher vielseitig anwendbar sein kann.

Das Gas kann zumindest eins aus Kohlenstoffmonoxid, Wasserstoff, Stickstoffdioxid, Kohlenstoffdioxid, und Methan aufweisen, so dass die Sensorvorrichtung vielseitig in verschiedensten Bereichen, beispielsweise für eine Brandschutzerkennung oder für eine Gebäudesicherung, verwendbar sein kann.

Insbesondere kann eine Mindestmenge des erfassten Gases etwa 10 ppm (engl. parts per million), insbesondere etwa 30 ppm, insbesondere etwa 50 ppm, insbesondere etwa 100 ppm, insbesondere etwa 300 ppm, insbesondere etwa 1000 ppm betragen, was für den zuletzt genannten Wert aufgrund eines internem Spannungshubs in dem Sensormodul einen ausgegebenen Spannungssignalwert von etwa 200 Millivolt (mV) entsprechen kann.

Das Energiespeichermodul kann zumindest eines aus einem Kondensator und einem Folien(-basierten)-Polymer-Akkumulator, aufweisen, so dass eine Ladung, die dem ausgegebenen und verstärkten elektrischen Signal zugeordnet ist, in dem Energiespeichermodul akkumuliert werden kann. Ferner kann die Sensorvorrichtung bei derartigen Energiespeichern kostengünstig ausgebildet sein, da diese Arten von Energiespeicher kostengünstige Bauteile sein können.

Insbesondere kann der Begriff "Folien-Polymer-Akkumulator" einen Energiespeicher in Form eines Akkumulators bezeichnen, der anstatt eines Elektrolyts eine oder mehrere Polymer-Folien aufweist. Beispielsweise kann der Folien-Polymer-Akkumulator als Lithium-Polymer-Akkumulator ausgebildet sein.

Insbesondere kann der Kondensator ein Dielektrikum zwischen seinen Kondensatorplatten aufweisen, das insbesondere aus einem Folienkunststoffmaterial, beispielsweise Polyester, Polyäthylenterephtalat und/oder Polypropylen, gefertigt sein kann. Der Kondensator kann aufgrund des Materials des Dielektrikums einen hohen Isolationswiderstand aufweisen, so dass Verluste der Ladung, die in dem Kondensator gespeichert werden kann, aufgrund von Selbstentladungseffekten des Kondensators minimiert werden können. Ferner kann der ESR (engl. equivalent series resistance)-Widerstand des Kondensators gering sein, so dass beim Entladevorgang des Kondensators ein unerwünschter Spannungsabfall an dessen internen Reihenwiderständen vermieden werden kann. Kondensatoren basierend auf Dielektrika in Form von Kunststofffolien können eine sehr hohe Impulsbelastbarkeit und relativ geringe interne ohmsche Widerstände aufweisen und zusätzlich besonders klein ausgestaltet sein, so dass die von den Kondensatoren bereitgestellte Kapazität zwar klein, aber ausreichend für die elektrische Versorgung des Sendemoduls sein kann.

Die Benachrichtigung kann zumindest ein Telegramm (oder eine Vielzahl von Telegrammen) aufweisen, so dass eine (zeitlich kontinuierliche) Fernmeldung über das Vorhandensein des Gases stattfinden kann. Insbesondere kann eine Anzahl von gesendeten Telegrammen auf einer Menge der gespeicherten Energie basieren.

Insbesondere kann die Benachrichtigung eine Alarmmeldung aufweisen, so dass ein Empfänger der Benachrichtigung über das Vorhandensein einer bestimmten Menge von Gas in der Umgebung der Sensorvorrichtung gewarnt werden kann.

Die Sensorvorrichtung kann als Brandschutzsensorvorrichtung ausgebildet sein.

Im Folgenden werden exemplarische Ausführungsbeispiele der vorliegenden Erfindung mit Verweis auf die folgenden Figuren detailliert beschrieben.
Fig. 1 ist ein Blockschaltbild, das eine Sensorvorrichtung zum Melden von vorhandenem Gas gemäß einem exemplarischen Ausführungsbeispiel der Erfindung zeigt.
Fig. 2 ist ein Blockschaltbild, das die Sensorvorrichtung in Fig. 1 während eines Betriebs zeigt.
Fig. 3 ist ein Blockschaltbild, das ein Ausführungsbeispiel eines Spannungsverstärkungsmoduls der Sensorvorrichtung in Fig. 1 zeigt.
Fig. 4 ist ein Blockschaltbild, das ein weiteres Ausführungsbeispiel des Spannungsverstärkungsmoduls der Sensorvorrichtung in Fig. 1 zeigt.
Fig. 5 ist ein Blockschaltbild, das ein noch weiteres Ausführungsbeispiel des Spannungsverstärkungsmoduls der Sensorvorrichtung in Fig. 1 zeigt.
Fig. 6 ist ein Diagramm, das ein an einem Sendemodul 108 der Sensoruchtung in Fig. 1 abfallende Spannungssignal in Abhängigkeit einer Betriebszeit der Sensorvorrichtung in Fig. 1 zeigt.

Die Darstellungen in den Figuren sind schematisch und nicht maßstabsgetreu. Gleiche oder ähnliche Komponenten oder Verfahrensschritte in unterschiedlichen Figuren sind mit gleichen Bezugszeichen versehen oder mit Bezugszeichen, deren erste Zahl sich unterscheidet.

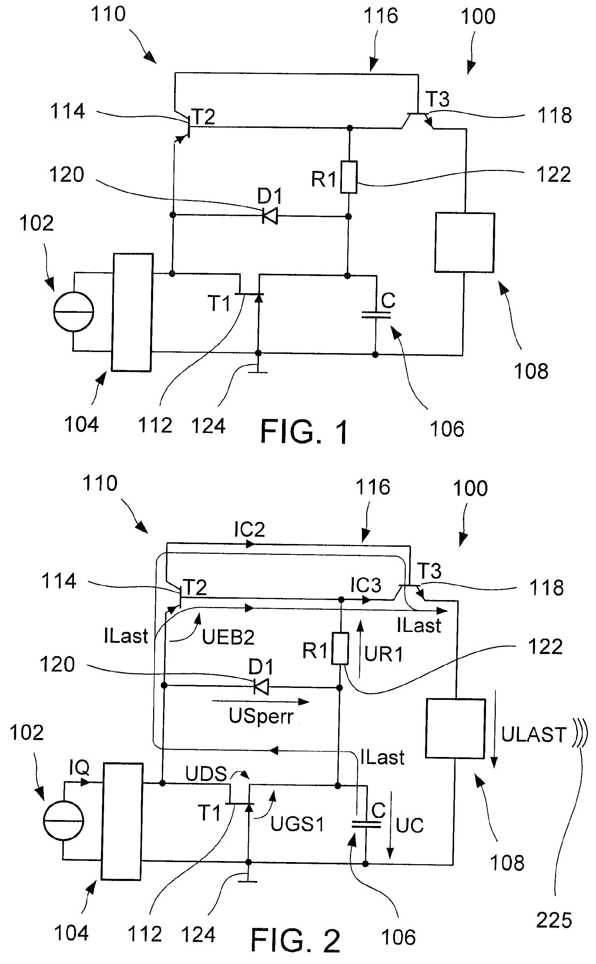

Fig. 1 zeigt eine Brandschutz-Sensorvorrichtung 100 zum Melden von vorhandenem Gas gemäß einem exemplarischen Ausführungsbeispiel der Erfindung. Die Sensorvorrichtung ist autark ausgebildet, so dass von der Sensorvorrichtung 100 erzeugter Strom (beziehungsweise Spannung) für einen Betrieb der Sensorvorrichtung 100 genügt und die Sensorvorrichtung 100 unabhängig von einer externen Stromquelle arbeitet. Ferner ist die Sensorvorrichtung 100 funkbasiert ausgebildet, so dass eine Alarmmeldung, die von der Sensorvorrichtung 100 erzeugt wird, unabhängig von einer Anwesenheit von Personen detektiert wird.

Zu diesem Zweck weist die Sensorvorrichtung 100 ein Sensormodul 102 zum Erfassen des vorhandenen Gases und zum Ausgeben eines Spannungssignals basierend auf dem erfassten Gas, ein Spannungsverstärkungsmodul 104 zum Verstärken des ausgegebenen Spannungssignals, ein Energiespeichermodul 106 zum Speichern von Energie, die auf dem ausgegebenen verstärktem Spannungssignal basiert, und ein Sendemodul 108 zum Senden einer Benachrichtigung basierend auf der gespeicherten Energie auf. Die Sensorvorrichtung 100 weist ferner ein Umschaltmodul 110 auf, das mit dem Spannungsverstärkungsmodul 104, dem Energiespeichermodul 106 und dem Sendemodul 108 verbunden und zum Umschalten zwischen einem Ladezustand und einem Entladezustand des Energiespeichermoduls 106 eingerichtet ist. Das Sensormodul 102, das Spannungsverstärkungsmodul 104 und das Energiespeichermodul 106 sind zueinander parallel geschaltet. Das Sendemodul 108 ist über das Umschaltmodul 110 mit dem Energiespeichermodul 106 verbunden.

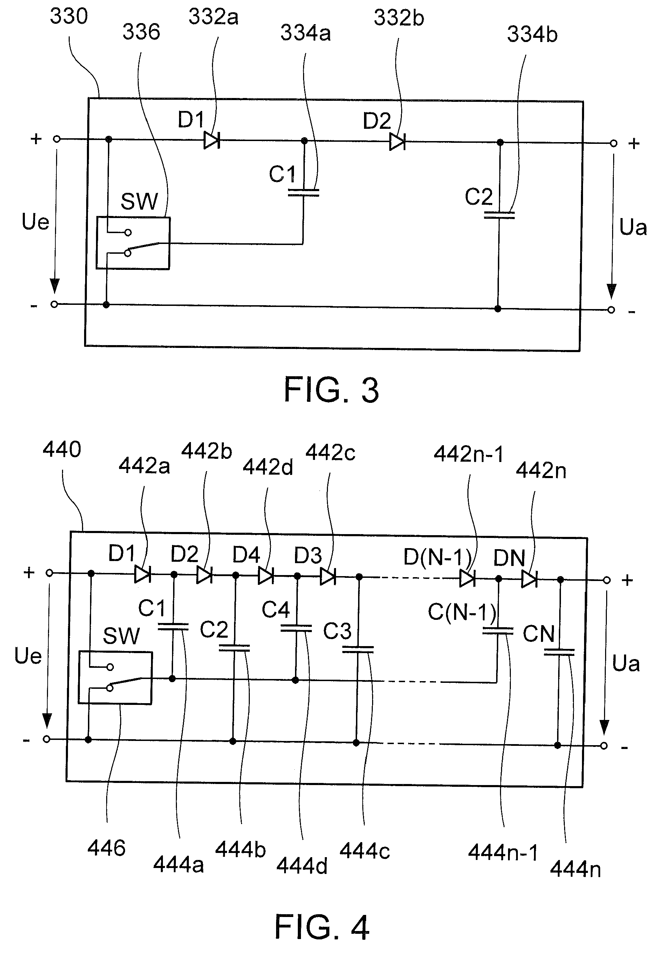

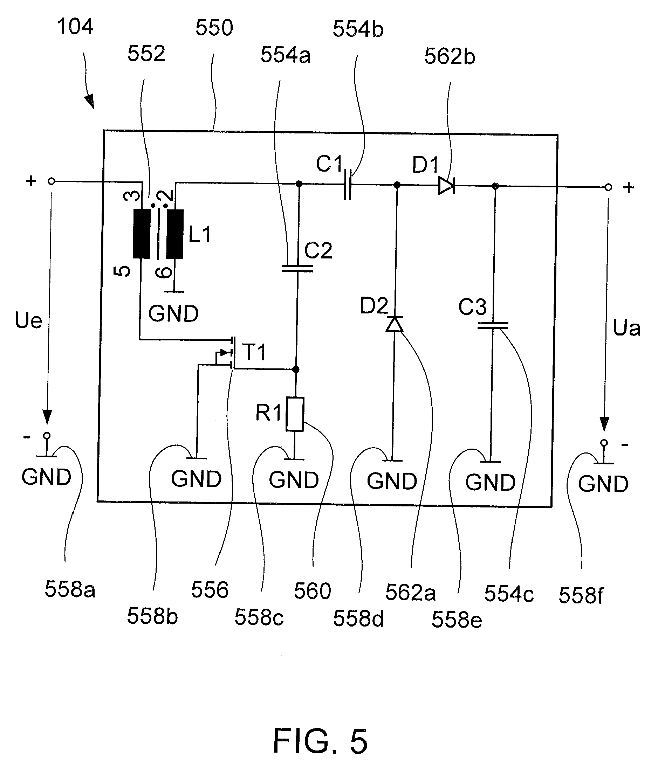

Das Sensormodul 102 ist als Kohlenmonoxid-Gassensor TGS5042 von Figaro Engineering Inc., Japan, ausgebildet, der einen elektrochemischen Kohlenmonoxid-Sensor mit einer Bauform einer handelsüblichen AA-Batterie aufweist. Das Sensormodul 102 kann Kohlenmonoxid-Konzentrationen zwischen 30 ppm und 10000 ppm erfassen. Das Spannungsverstärkungsmodul 104 ist als Ladungspumpe (Fig. 3, 4) oder als Spannungsaufwärtswandler (Fig. 5) ausgebildet. Das Energiespeichermodul 106 weist einen Kondensator auf, der eine Ladungsspeicherung einer gewissen Kapazität (gemessen in Mikro-Farad (µF)) ermöglicht und als 2 Mal 10 µF Kondensator vom Typ MKT10/100 V von Epcos ausgebildet ist. Ein Material einer Kunststofffolie, die zwischen Kondensatorplatten des Kondensators 106 angeordnet ist und als Dielektrikum wirkt, weist Polyester auf. Die nachfolgende Tabelle gibt eine Übersicht über weitere Ausführungsbeispiele des Kondensators 106.

| Typ | Kapazität | Spannung | Dielektrikum |
|---|---|---|---|
| Epcos MKP | 15 / 22 / 30 µF | 450V | Polypropylen |
| Epcos MKT | 4,7 / 10 µF | 100V | Polyäthylenterephtalat |
| WIMA MKS4 | 1 µF | 400V | Polyester |
| WIMA MKC4 | 1,5 µF | 400V | Polyester |
| Vishay/Roederstein MKT1813 | 6,8 µF | 100V | Polyester |
| Vishay MKT1822 | 2,2 / 10 µF | 63V | Polyester |

Das Sendemodul 108 ist als STM 300 Funkmodul von EnOcean, Deutschland, ausgebildet und sendet in einem 868 Mega-Herz bzw. 315MHz (MHz) Frequenzband. Das Sendemodul 108 sendet Benachrichtigungen mit einer Datenübertragungsrate von 125 Kilo-Bit/Sekunde aus, die als Telegramme einer Dauer von einer Millisekunde (ms) ausgebildet sind. Die ausgesendeten Telegramme werden innerhalb von 30 ms mehrmals wiederholt, um ein Auftreten von Übertragungsfehlern zu vermeiden. Eine Reichweite des Sendemoduls 108 beträgt etwa 300 Meter (m) im unbebautem Gelände und bis zu 30 m in Gebäuden. In den Telegrammen wird eine 32-Bit-Identifikationsnummer oder 16-Bit virtuelle Identifikationsnummer des jeweiligen Sendemoduls 108 mitübertragen, so dass eine Fehlzuordnung von empfangenden Telegrammen bezüglich anderer, gleichartig ausgebildeten Sendemodule vermieden wird.

Das Umschaltmodul 110 weist einen n-Kanal-Sperrschicht-Feldeffekttransistor (engl. Junction Field Effect Transistor JFET) 112 vom Typ BF245C von Philips Semiconductors auf, der in Reihe zwischen dem Spannungsverstärkungsmodul 104 und dem Kondensator 106 geschaltet ist. Der Transistor 112 ist in Fig. 1 mit T1 bezeichnet. Ein Gate-Anschluss des Transistors 112 ist über eine Ladung des Kondensators 106 steuerbar. Ein Drain-Anschluss des Transistors 112 ist mit einem Emitter-Anschluss eines ersten pnp-Bipolar-Transistors 114 vom Typ 2N3906 von ST Microelectronics verbunden, der ein Bauteil einer Thyristortetrode 116 des Umschaltmoduls 112 ist. Ein zweiter npn-Bipolar-Transistor 118 Typ 2N3904 von ST Microelectronics, der ein weiteres Bauteil der Thyristortetrode 116 ist, ist über seinen Emitter-Anschluss mit dem Sendemodul 108 verbunden. Ein Kollektor-Anschluss des ersten Transistors 114 ist mit einem Basis-Anschluss des zweiten Transistors 118 verbunden, und ein Basis-Anschluss des ersten Transistors 114 ist mit einem Kollektor-Anschluss des zweiten Transistors 118 verbunden. Der erste Transistor 114 ist in Fig. 1 mit T2 und der zweite Transistor 118 ist in Fig. 1 mit T3 bezeichnet. Der Emitter-Anschluss des ersten Transistor 114 bildet eine Anode der Thyristortretrode 116 und der Emitter-Anschluss des zweiten Transistors 118 bildet eine Kathode der Thyristortretrode 118. Eine Diode 120 des Umschaltmoduls 110 ist parallel zu dem Transistor 112 geschaltet und mit dem Thyristor 116 verbunden. Die Diode 112 ist als Standarddiode vom Typ 1N4148 von Vishay Semiconductors ausgebildet und in Fig. 1 mit D1 bezeichnet. Ein 470 Kilo-Ohm (kΩ)-Drahtwiderstand 122 verbindet den Kondensator 106, den Transistor 112, die Diode 120 und eine Signalleitung miteinander, die als Basis-Kollektor-Strecke den ersten Transistor 114 mit dem zweiten Transistor 118 des Thyristors 116 verbindet. Der Widerstand 122 ist in Fig. 1 mit R1 bezeichnet. Da das Umschaltmodul 110 einen geringen Stromverbrauch während eines Betriebs aufweist, kann das Sendemodul 108 mittels durch das Sensormodul 102 erzeugter Spannung betrieben werden.

Die Sensorvorrichtung 110 ist ferner mittels eines gemeinsamen Masseanschlusses 124 miteinander verbunden, der zwischen dem Spannungsverstärkungsmodul 104 und dem Kondensator 106 in einer den Transistor T1 nicht aufweisenden Signalleitung angeordnet.

Das Spannungsverstärkungsmodul 104, der Kondensator 106, das Sendemodul 108 und das Umschaltmodul 110 sind auf einem gemeinsamen Substrat als integrierte Schaltung ausgebildet. Das Sensormodul 102 ist in entsprechende Anschlüsse, die auf der integrierten Schaltung gebildet sind, einsteckbar und kann gegen ein gleichartiges Sensormodul oder gegen andere Sensormodule ausgetauscht werden, die dazu eigerichtet sind, ein anderes Gas oder Gasgemisch zu erfassen.

Ein Empfangsmodul zum Empfangen der von dem Sendemodul 108 ausgesendeten Telegramme ist als EVA 300-2 Modul in Kombination mit einem TCM 300 Funkmodul von EnOcean ausgebildet und über eine USB-Verbindung mit einem Computer verbunden. Die Telegramme sind unter Verwendung der Software "WinEtel" von EnOcean an einem Bildschirm des Computers darstellbar.

Mit Bezug auf Fig. 2 wird ein Betrieb der Sensorvorrichtung 100 erläutert. Zu Beginn des Betriebs der Sensorvorrichtung 100 ist die Sensorvorrichtung strom- beziehungsweise spannungslos, und der Kondensator 106 der Sensorvorrichtung 100 ist entladen.

Sobald das Sensormodul 102 Kohlenmonoxid in der Umgebung des Sensormoduls 102 erfasst, erzeugt das Sensormodul 102 ein Spannungssignal, das als Strom IQ ausgegeben wird. Das Spannungsverstärkungsmodul 104 empfängt den ausgegebenen Strom IQ in Form von Ladung, sammelt diese an und verstärkt beziehungsweise schaukelt die Ausgangsspannung auf, die dem Transistor 112, dem ersten Transistor 114 der Thyristortetrode 116 und der in Sperrrichtung geschaltete Diode 120 zugeführt wird. Die verstärkte Spannung, die an einem Drain-Anschluss des Transistors 112 anliegt, erzeugt einen Ladungsfluss in einem leitenden Kanal des Transistors 112, so dass ein entsprechender Strom über einen Source-Anschluss des Transistors 112 dem Kondensator 106 zugeführt wird. Die in den Kondensator 106 akkumulierte Ladung erzeugt eine Spannung UC am Kondensator 106. Dieser Spanungszuwachs entspricht einem Ladevorgang des Kondensators 106. Erreicht der Transistor 112 bei genügend großer Drain-Source-Spannung UDS seinen Abschnürbereich, ist der Drainstrom nur noch von der Gate-Source-Spannung UGS1 abhängig, die der am Kondensator 106 anliegenden Spannung UC entspricht (UC = -UGS1). Bei der Einschnürspannung UGSein sperrt der Transistor 112 den leitenden Kanal, so dass dem Kondensator 106 keine Ladung mehr zugeführt wird.

Da sich die Transistoren 114, 118 der Thyristortetrode 116 bei Betriebsbeginn in einem gesperrten Zustand befinden, kann der Strom IQ während des Ladevorgangs des Kondensators 106 nur durch den Transistor 112 fließen, solange der Drain-Source-Strom des Transistors 112 nicht von der Gate-Source-Spannung UGS1 abgeschnürt wird. Sobald der Transistor 120 den Drain-Source-Strom abzuschnüren beginnt, entsteht an den Drain-Source-Anschlüssen des Transistors 112 die Drain-Source-Spannung UDS, die über den Widerstand 122 ebenfalls an der Verbindung zwischen dem Emitter-Anschluss und dem Basis-Anschluss des Transistors 114 (beziehungsweise an einer Emitter-Basis-Strecke des Transistors 114) anliegt (in diesem Fall gilt UDS = UEB2 - UR1, wobei UR1 die am Widerstand 122 abfallende Spannung bezeichnet). Sobald die Sperrspannung USperr der Diode 120 die Spanungssumme bestehend aus Diffusionsspannung des ersten Transistors 114 der Thyristortetrode 116 und der Abfallspannung UR1 der Widerstandes 122 erreicht, öffnet sich der leitende Kanal des ersten Transistors 114, so dass ein Strom IC2 zur Basis des zweiten Transistors 118 fließt. Folglich schaltet der zweite Transistor 118 der Thyristortetrode 116 durch und ermöglicht, dass ein Strom IC3 zum Kollektor-Anschluss des ersten Transistors 114 fließt und den ersten Transistor 114 in einem leitenden Zustand hält. Da der Kollektor-Anschluss des ersten Transistors 114 mit dem Basis-Anschluss des zweiten Transistors 118 und der Kollektor-Anschluss des zweiten Transistors 118 mit dem Basis-Anschluss des ersten Transistors 114 verbunden ist, halten sich die ersten und zweiten Transistoren 114, 118 der Thyristortetrode 116 gegenseitig in einem leitenden Zustand. Das beschriebene Anschalten des ersten und zweiten Transistors 114, 118 der Thyristortetrode 116 erfolgt schlagartig, und es fließt ein Strom ILast über der Diode 120, den ersten Transistor 114 der Thyristortetrode 116 und den zweiten Transistor 118 der Thyristortetrode 116 zu dem Sendemodul 108. Der Laststrom ILast fließt solange, bis der Kondensator 106 entladen ist. Dieser Vorgang wird als Entladezustand des Kondensators bezeichnet.

Eine Spannung ULast, die aufgrund des zugeführten Laststroms ILast am Sendemodul 108 abfällt, ermöglicht die Aussendung eines oder mehrerer Telegramme, die in Fig. 2 mittels Funklinien dargestellt und mit dem Bezugszeichen 225 versehen sind. Eine Anzahl der gesendeten Telegramme 225 hängt von dem Wert der gespeicherten Ladung in dem Energiespeichermodul 106 im Vergleich zum Leistungsverbrauch des Sendemoduls 108 für das Erzeugen und Aussenden eines Telegramms 225 ab.

Wenn der Laststrom ILast abbricht, sperrt die Thyristortetrode 116. Folglich fließt der Strom IQ durch den Transistor 112 zu dem Kondensator 106, der sich wieder auflädt, und die Versorgung des Sendemoduls 108 mit elektrischer Energie beginnt erneut.

Im Folgenden werden Ausführungsbeispiele des Spannungsverstärkungsmoduls 104 der Sensorvorrichtung 100 mit Bezug auf die Fig. 3 bis 5 erklärt.

Fig. 3 zeigt ein Spannungsverstärkungsmodul 104, das als Ladungspumpe 330 ausgebildet ist. Die Ladungspumpe 330 weist eine erste Diode 332a, eine zweite Diode 332b, einen Pumpkondensator 334a, einen Ladekondensator 334b und ein Schaltelement 336 in Form eines Komplementärer-Metalloxidhalbleiter (CMOS)-Inverterelements auf. Das CMOS-Inverterelement 336 weist einen n-Kanal und einen p-Kanal Metalloxidhalbleiter-Feldeffekttransistor (engl. metal oxide semiconductor field effect transistor MOSFET) auf, die auf einem gemeinsamen Substrat angeordnet sind. Die erste und zweite Diode 332a, b sind identisch ausgebildet und in Reihe geschaltet, und der Pumpkondensator 334a und der Ladekondensator 334b sind identisch ausgebildet und zueinander parallel über die zweite Diode 332b und das Schaltelement 336 geschaltet. Das Schaltelement 336 ist parallel zu dem Sendemodul 108 geschaltet. Ferner ist der Pumpkondensator 334a mit einem Knoten in einer Signalleitung verbunden, die die erste Diode 332a und die zweite Diode 332b miteinander verbindet.

In einem Betrieb der Ladungspumpe 330 entspricht eine Eingangsspannung Ue der Ladungspumpe 330 der vom Sensormodul 102 erzeugten Spannung. Das Schaltelement 336 der Ladungspumpe 336 schaltet periodisch mit einer Schaltfrequenz zwischen zwei Schaltzuständen um. In einem ersten Schaltzustand, der in Fig. 3 dargestellt ist, verbindet das Schaltelement 336 den Pumpkondensator 334a mit dem Ladekondensator 334b. Folglich lädt sich der Pumpkondensator 334a über die Diode 332a auf. Danach schaltet das Schaltelement in einen zweiten Schaltzustand um, in dem der Pumpkondensator 334a mit einem Knoten einer Signalleitung zwischen dem Sensormodul 102 und der ersten Diode 332a verbunden ist. In diesem Schaltzustand sind die Eingangsspannung Ue und die Spannung an dem Kondensator 334a in Serie geschaltet, so dass die Diode 332a sperrt und die Diode 332b von einem Sperrzustand in einen leitenden Zustand übergeht. Folglich wird der Kondensator 334b auf eine Ausgangsspannung Ua aufgeladen, die idealerweise ohne weitere Ohmsche Verluste die doppelte Eingangsspannung Ue abzüglich der doppelten Spannung beträgt, die an den Dioden 332a, b abfällt. Danach schaltet das Schaltelement 336 in seinen ersten Schaltzustand und das Laden des Pumpkondensators 334a beginnt erneut.

Fig. 4 zeigt ein weiteres Ausführungsbeispiel des Spannungsverstärkungsmoduls 104 der Sensorvorrichtung 100 in Fig. 1, das als kaskadenartige Ladungspumpe 440 ausgebildet ist, die eine Vervielfachung einer Eingangsspannung Ue ermöglicht, die der durch das Sensormodul 102 erzeugten Spannung entspricht. Die Ladungspumpe 440 ist ähnlich zu der Ladungspumpe 330 ausgebildet und weist n identische Dioden 442a-n, n identische Kondensatoren 444a-n und ein Schaltelement 446 in Form eines CMOS-Inverterelements 446 auf. Die Dioden 442a-n sind in Serie geschaltet, und eine erste Diode 442a der n Dioden 442a-n ist mit dem Sensormodul 102 verbunden. Jeder der Kondensatoren 444a-n ist parallel zu einem in Stromflussrichtung gesehen stromabwärts übernächsten, parallel geschalteten Kondensator 444a-n geschaltet. Die Kondensatoren 444a-(n-1) dienen als Pumpkondensatoren und der Kondensator 444n dient als Ladekondensator, der zum Ausgeben einer Ausgabespannung Ua eingerichtet ist. Der erster Kondensator 444a ist stromabwärts des Sensormoduls 102 angeordnet und mit einem Knoten in einer Signalleitung, die die erste Diode 442a und die zweite Diode 442b verbindet, und mit dem Schaltelement 446 verbunden. Jeder bezüglich des ersten Kondensators 444a als übernächstes parallel geschaltete Kondensator 444d, ..., 444n-1 ist mit entsprechenden Knoten in Signalleitungen, die jeweils zwei Dioden 442a-n miteinander verbinden, und mit dem Schaltelement 446 verbunden. Der stromabwärts des Sensormoduls 102 als zweites angeordnete Kondensator 444b und jeder bezüglich des zweiten Kondensators 444b zyklisch stromabwärts übernächste Kondensator 444d, ..., 444n ist mit einem entsprechenden Knoten in einer Signalleitung verbunden, die zwei Dioden 442b, 444c, 4442, ..., 444n miteinander verbinden, so dass diese Kondensatoren 444b, 444c, ... parallel zum Ladekondensator 444n geschaltet sind.

Ein Betrieb der Ladungspumpe 440 ist ähnlich zu einem Betrieb der Ladungspumpe 330. Die Kondensatoren 440a, 444d, ..., 444n-1 werden in dem ersten Schaltzustand des Schaltelements 446 aufgeladen, so dass in dem zweiten Schaltzustand des Schaltelements 446 die Ausgabespannung an den Ladekondensator 444n mittels der Kondensatoren 444b, 444c, ... ausgegeben wird. Die von der Ladungspumpe 444 erzeugte Ausgangsspannung Ua beträgt (idealerweise) die n-1-fache Eingangsspannung Ua abzüglich der n-fachen Spannung, die an einer der Diode 442a-n abfällt.

Fig. 5 zeigt ein weiteres Ausführungsbeispiel des Spannungsverstärkungsmoduls 104, das als Aufwärtswandler 550 ausgebildet ist. Der Aufwärtswandler 550 ist ein Teilbauteil eines Spanungswandlers vom Typ LTC3108 von Linear Technology, Kalifornien, USA und weist einen Transformator 552 und Kondensatoren 554a-c auf, die über ein als MOSFET ausgebildetes Schaltelement 556 resonant miteinander verschaltet sind. Die individuellen Elemente des Aufwärtswandlers 550 sind mit entsprechenden Masse-Anschlüssen 558a-f verbunden. Die Eingangsspannung Ue wird bezüglich eines Masseanschluss 558a bestimmt. Eine Primärspule des Transformators 552 ist über das Schaltelement 556 mit der Masse 558b und eine Sekundärspule des Transformators 552 ist mit dem Kondensator 554b verbunden. Ein Source-Anschluss des MOSFETs 556 ist mit einem Masseanschluss 558b verbunden. Ein Gate-Anschluss des Schaltelements 556 ist über einen Widerstand 560 mit einem Massenanschluss 558c und mit dem Kondensator 554a verbunden. Eine Diode 562a ist ausgangseitig des Kondensators 554b verbunden und parallel zu den Kondensatoren 554a, 554c geschaltet. Der Kondensator 554a ist mit einer Signalleitung verbunden, die die Sekundärspule des Transformators 552 mit dem Kondensator 554b miteinander verbindet. Eine Diode 562b verbindet die Kondensatoren 554b und 554c miteinander. Die Diode 562a beziehungsweise der Kondensator 554c sind mit einem Masseanschluss 558d beziehungsweise 558e verbunden. Die Ausgangsspannung Ua wird bezüglich eines Masseanschluss 558f bestimmt.

Der Kondensator 554a in Zusammenhang mit dem Widerstand 560 dient als RC Glied zur zeitlichen Ansteuerung des Gate-Anschlusses des Transistors 556, der permanent die Primärspule des Transformators mit der Masse 558b verbindet. Durch die zeitliche Unterbrechung des Stromflusses an der Primärspule wird eine Induktionsspannung an der Primärspule des Transformators 552 erzeugt, die sich entsprechend dem Induktionsgesetz auch auf die Sekundärspule des Transformators 552 überträgt. Die induzierte Spannung in der Sekundärspule ist höher als die Eingansspannung Ue. Der Kopplungskondensator 554b ist dazu eingerichtet, diese induzierte Spannung zum Speicherkondensator 554c zu übertragen. Die Dioden 562a, b sind dazu eingerichtet, einen von dem Transformator 552 erzeugten Strom gleichzurichten. Der Speicherkondensator 554c summiert als Ladekondensator die verstärkte, gleichgerichtete Spannung auf.

In einem Betrieb des Aufwärtswandlers 550 fällt die Eingangsspannung Ue an dem Transformator 552 ab, so dass ein entsprechender Strom in Primär- und Sekundärspulen des Transformators 552 erzeugt wird. Der in einer Sekundärspule des Transformators 552 erzeugte Strom wird dem Kopplungskondensator 554b zugeführt. Durch geeignetes zeitliches Schalten des Schaltelements 556 wird ein in der Primärspule des Transformators 552 erzeugte Induktionsspannung auf die Sekundärspule übertragen. Die in der Sekundärspule induzierte Spannung erzeugt einen Strom, der über den Kopplungskondensator 554b mittels der Dioden 562a, b gleichgerichtet und in dem Ladekondensator 554c als Ladung aufgesammelt und als Ausgangsspannung Ua ausgegeben wird.

Für eine detaillierte Darstellung eines Aufbaus und einer Funktionsweise des Aufwärtswandlers wird auf "LTC3108, UltraLow Volateg Step-Up Converter and Power Manager" von Linear Technology, 2010, verwiesen, dessen Inhalt durch Bezugnahme vollinhaltlich aufgenommen wird.

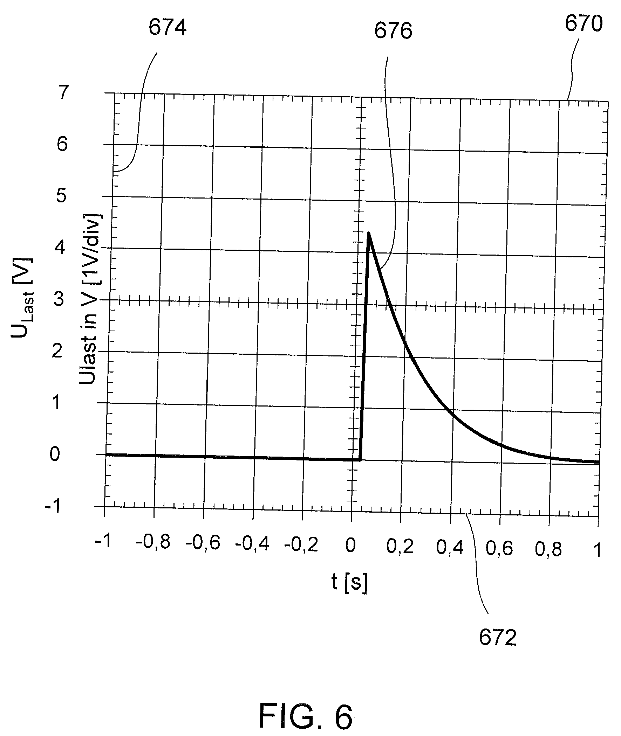

Fig. 6 zeigt ein Diagramm 670, das eine Zeitabhängigkeit der Lastspannung ULast am Sendemodul 108 der Sensorvorrichtung 100 in Fig. 1 zeigt. Eine Ordinate 672 des Diagramms 670 zeigt die Betriebszeit der Sensorvorrichtung in Sekunden (s). Eine Abszisse 674 des Diagramms 670 zeigt die Lastspannung ULast in Volt (V). Eine Kurve 676 der Lastspannung ULast zeigt den schlagartigen Anstieg der Lastspannung auf ungefähr 4,2 V innerhalb von 40 Millisekunden (ms) und eine exponentielle Abnahme der Lastspannung ULast in einer Zeitdauer von ungefähr einer Sekunde. Diese Abnahme wird durch den Entladevorgang des Kondensators 106 verursacht.

Ergänzend ist darauf hinzuweisen, dass "aufweisend" keine anderen Elemente oder Schritte ausschließt und die Verwendung des Artikels "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Sensorvorrichtung (100) zum Melden von vorhandenem Gas, wobei die Sensorvorrichtung (100) aufweist:
- ein Sensormodul (102) zum Erfassen des vorhandenen Gases und zum Ausgeben eines elektrischen Signals basierend auf dem erfassten Gas,
- ein Signalverstärkungsmodul (104) zum Verstärken des ausgegebenen elektrischen Signals,
**dadurch gekennzeichnet, dass**
die Sensorvorrichtung (100) aufweist:
- ein Energiespeichermodul (106) zum Speichern von Energie, die auf dem verstärkten elektrischen Signal basiert,
- ein Sendemodul (108) zum Senden einer Benachrichtigung (225) basierend auf der gespeicherten Energie des Energiespeichermoduls (106).

2. Sensorvorrichtung (100) nach Anspruch 1, wobei das Sensormodul (102), das Signalverstärkungsmodul (104) und das Sendemodul (108) parallel zueinander geschalt sind.

3. Sensorvorrichtung (100) nach Anspruch 1 oder 2, wobei die Sensorvorrichtung (100) ferner aufweist:
- ein Umschaltmodul (110), das mit dem Signalverstärkungsmodul (104), dem Energiespeichermodul (106) und dem Sendemodul (108) verbunden und zum Umschalten zwischen einem Ladezustand und einem Entladezustand des Energiespeichermoduls (106) eingerichtet ist.

4. Sensorvorrichtung (100) nach Anspruch 3, wobei das Umschaltmodul (110) einen Transistor (112) und einen Thyristor (116) aufweist, wobei eine Anode des Thyristors (116) über den Transistor (112) mit dem Energiespeichermodul (106) und eine Kathode des Thyristors (116) mit dem Sendemodul (108) verbunden ist.

5. Sensorvorrichtung (100) nach Anspruch 4, wobei das Umschaltmodul (110) eine Diode (120) aufweist, die parallel zu dem Transistor (112) und in Sperrrichtung geschaltet ist.

6. Sensorvorrichtung (100) nach Anspruch 4 oder 5, wobei das Umschaltmodul (110) einen Widerstand (122) aufweist, der zwischen dem Transistor (112) und dem Thyristor (116) angeordnet ist.

7. Sensorvorrichtung (100) nach einem der Ansprüche 1 bis 6, wobei das Signalverstärkungsmodul (104) als Ladungspumpe (330, 440) oder als Spannungsaufwärtswandler (550) ausgebildet ist.

8. Sensorvorrichtung (100) nach einem der Ansprüche 1 bis 7, wobei das Signalverstärkungsmodul (104) einen Transformator (552), einen Kondensator (554c) und einen Transistor (556) aufweist, die resonant miteinander geschaltet sind.

9. Sensorvorrichtung (100) nach einem der Ansprüche 1 bis 8, wobei das Sensormodul (102) ein chemisches Sensorelement aufweist.

10. Sensorvorrichtung (100) nach einem der Ansprüche 1 bis 9, wobei das Sensormodul (102) eine Mehrzahl von chemischen Sensorelementen aufweist.

11. Sensorvorrichtung (100) nach einem der Ansprüche 1 bis 10, wobei das Gas als für ein Lebewesen toxisches Gas ausgebildet ist.

12. Sensorvorrichtung (100) nach einem der Ansprüche 1 bis 11, wobei das Gas zumindest eins aus Kohlenstoffmonoxid, Wasserstoff, Kohlenstoffdioxid, Stickstoffdioxid und Methan aufweist.

13. Sensorvorrichtung nach einem der Ansprüche 1 bis 12, wobei das Energiespeichermodul (106) zumindest eins aus einem Kondensator und einem Folien-Polymer-Akkumulator aufweist.

14. Sensorvorrichtung (100) nach einem der Ansprüche 1 bis 13, wobei die Benachrichtigung (225) zumindest ein Telegramm (225) aufweist.

15. Verfahren zum Melden von vorhandenem Gas, wobei das Verfahren aufweist:
- Erfassen des vorhandenen Gases und Ausgeben eines elektrischen Signals basierend auf dem erfassten Gas mittels eines Sensormoduls (102),
- Verstärken des elektrischen Signals mittels eines Signalverstärkungsmoduls (104),
**dadurch gekennzeichnet, dass**
das Verfahren aufweist:
- Speichern einer Energie, die auf dem verstärkten elektrischen Signal basiert, mittels eines Energiespeichermoduls (106), und
- Senden einer Benachrichtigung (225) mittels eines Sendemoduls (108) basierend auf der gespeicherten Energie des Energiespeichermoduls (106).

## Claims

1. A sensor device (100) for reporting the presence of gas, the sensor device (100) comprising:
- a sensor module (102) for detecting the presence of gas and for outputting an electrical signal based on the detected gas;
- a signal amplification module (104) for amplifying the output electrical signal, **characterized in that**
the sensor device (100) comprises:
- an energy storage module (106) for storing energy that is based on the amplified electrical signal;
- a transmitter module (108) for transmitting a notification (225) based on the stored energy of the energy storage module (106).

2. The sensor device (100) according to claim 1, wherein the sensor module (102), the signal amplification module (104) and the transmitter module (108) are connected in parallel to each other.

3. The sensor device (100) according to claim 1 or 2, the sensor device (100) further comprising:
- a switching module (110), which is connected to the signal amplification module (104), the energy storage module (106) and the transmitter module (108) and configured to switch between a charge state and a discharge state of the energy storage module (106).

4. The sensor device (100) according to claim 3, wherein the switching module (110) comprises a transistor (112) and a thyristor (116), an anode of the thyristor (116) being connected by way of the transistor (112) to the energy storage module (106), and a cathode of the thyristor (116) being connected to the transmitter module (108).

5. The sensor device (100) according to claim 4, wherein the switching module (110) comprises a diode (120), which is connected in parallel to the transistor (112) and in the reverse direction.

6. The sensor device (100) according to claim 4 or 5, wherein the switching module (110) comprises a resistor (122), which is disposed between the transistor (112) and the thyristor (116).

7. The sensor device (100) according to any one of claims 1 to 6, wherein the signal amplification module (104) is designed as a charge pump (330, 440) or as a step-up voltage converter (550).

8. The sensor device (100) according to any one of claims 1 to 7, wherein the signal amplification module (104) comprises a transformer (552), a capacitor (554c) and a transistor (556), which are resonantly connected to each other.

9. The sensor device (100) according to any one of claims 1 to 8, wherein the sensor module (102) comprises a chemical sensor element.

10. The sensor device (100) according to any one of claims 1 to 9, wherein the sensor module (102) comprises a plurality of chemical sensor elements.

11. The sensor device (100) according to any one of claims 1 to 10, wherein the gas is a toxic gas for living beings.

12. The sensor device (100) according to any one of claims 1 to 11, wherein the gas comprises at least one carbon monoxide, hydrogen, carbon dioxide, nitrogen oxide and methane.

13. The sensor device according to any one of claims 1 to 12, wherein the energy storage module ((106) comprises at least one of a capacitor and a rechargeable film polymer battery.

14. The sensor device (100) according to any one of claims 1 to 13, wherein the notification (225) comprises at least one frame (225).

15. A method for reporting the presence of gas, the method comprising the following steps:
- detecting the presence of gas and outputting an electrical signal based on the detected gas by way of a sensor module (102);
- amplifying the electrical signal by way of a signal amplification module (104), **characterized in that**
the method comprises:
- storing energy that is based on the amplified electrical signal, by way of an energy storage module (106); and
- transmitting a notification (225) by way of a transmitter module (108) based on the stored energy of the energy storage module (106).

## Revendications

1. Dispositif capteur (100) pour alerter de la présence de gaz, où le dispositif capteur (100) présente :
- un module capteur (102) pour la détection de la présence de gaz et pour la délivrance d'un signal électrique basé sur le gaz détecté,
- un module d'amplification de signal (104) pour l'amplification du signal électrique émis,
**caractérisé en ce que**
le dispositif détecteur (100) présente :
- un module d'accumulation d'énergie (106) pour le stockage d'énergie, qui est basée sur le signal électrique amplifié,
- un module de transmission (108) pour la transmission d'une information (225) se basant sur l'énergie emmagasinée du module d'accumulation d'énergie (106).

2. Dispositif capteur (100) selon la revendication 1, dans lequel le module capteur (102), le module d'amplification de signal (104) et le module de transmission (108) sont branchés en parallèle les uns par rapport aux autres.

3. Dispositif capteur (100) selon la revendication 1, ou 2, où le dispositif capteur (100) présente en outre :
- un module de commutation (110), qui est relié avec le module d'amplification de signal (104), le module d'accumulation d'énergie (106) et le module de transmission (108) et qui est conçu commuter entre un état de charge et un état de décharge du module d'accumulation d'énergie (106).

4. Dispositif capteur (100) selon la revendication 3, dans lequel le module de commutation (110) présente un transistor (112) et un thyristor (116), où une anode du thyristor (116) est reliée avec le module d'accumulation d'énergie (106) par l'intermédiaire du transistor (112) et une cathode du thyristor (116) est reliée avec le module de transmission (108).

5. Dispositif capteur (100) selon la revendication 4, dans lequel le module de commutation (110) présente une diode (120), qui est branchée en parallèle par rapport au transistor (112) et en direction inverse.

6. Dispositif capteur (100) selon la revendication 4, ou 5, dans lequel le module de commutation (110) présente une résistance (122) qui est agencée entre le transistor (112) et le thyristor(116).

7. Dispositif capteur (100) selon l'une des revendications 1 à 6, dans lequel le module d'amplification de signal (104) est conçu sous la forme d'une pompe de charge (330, 440) ou d'un convertisseur élévateur de tension (550).

8. Dispositif capteur (100) selon l'une des revendications 1 à 7, dans lequel le module d'amplification de signal (104) présente un transformateur (552), un condensateur (554c) et un transistor (556), qui sont branchés ensemble en résonance.

9. Dispositif capteur (100) selon l'une des revendications 1 à 8, dans lequel le module capteur (102) présente un élément capteur chimique.

10. Dispositif capteur (100) selon l'une des revendications 1 à 9, dans lequel le module capteur (102) présente une multitude d'éléments capteurs chimiques.

11. Dispositif capteur (100) selon l'une des revendications 1 à 10, dans lequel le gaz est un gaz toxique pour un être vivant.

12. Dispositif capteur (100) selon l'une des revendications 1 à 11, dans lequel le,gaz présente au moins un gaz parmi le monoxyde de carbone, l'hydrogène, le dioxyde de carbone, le dioxyde d'azote et le méthane.

13. Dispositif capteur (100) selon l'une des revendications 1 à 12, dans lequel le module d'accumulation d'énergie (106) présente au moins un élément parmi un condensateur et un accumulateur en feuilles-polymère.

14. Dispositif capteur (100) selon l'une des revendications 1 à 13, dans lequel l'information (225) présente au moins un télégramme (225).

15. Procédé d'alerte de présence de gaz, où le procédé présente :
- une détection du gaz présent et une émission d'un signal électrique basé sur le gaz détecté au moyen d'un module capteur (102),
- l'amplification du signal électrique au moyen d'un module d'amplification de signal (104),
**caractérisé en ce que**
le procédé présente :
- une accumulation d'énergie qui est basée sur le signal électrique amplifié au moyen d'un module d'accumulation d'énergie (106), et
- la transmission d'une information (225) au moyen d'un module de transmission (108) basée sur l'énergie accumulée du module d'accumulation d'énergie (106).
